# EUROPEAN PATENT APPLICATION

(11) **EP 3 398 514 A1**
(43) Date of publication of application: **07.11.2018**
(21) Application number: 17169059.7
(22) Date of filing: 02.05.2017
(51) Int. Cl.: A61B 6/00, G06F 19/00

(54) **X-RAY SYSTEM FOR GUIDED OPERATION**

(71) Applicant: Koninklijke Philips N.V., 5656 AE Eindhoven (NL)
(72) Inventor: VOGTMEIER, Gereon, 5656 AE Eindhoven (NL); CHAKRABARTI, Biswaroop, 5656 AE Eindhoven (NL); RAGHOTHAM VENKAT, Prasad, 5656 AE Eindhoven (NL); YOUNG, Stewart, 5656 AE Eindhoven (NL)
(74) Representative: de Haan, Poul Erik

(57) **Abstract**

The present invention relates to an X-ray device, an X-ray system and a method of operation thereof. An operator and a patient are identified by an ID-system comprising one or more ID-means. The operator expertise level is determined. Image acquisition for generating an X-ray image is performed by using an interface for adaptive interaction of the operator and X-ray device. The interface is adapted for outputting at least one option selector. At least one subsequent option selector is adapted according to the operator expertise level and/or object data.

## Description

### FIELD OF THE INVENTION

The present invention relates to X-ray systems and devices and the operation thereof.

### BACKGROUND OF THE INVENTION

X-ray systems are normally operated by experts in the field of image acquisition using X-rays.

X-ray systems are an attractive solution for rural areas but also for areas with high population density in such cases where additional diagnostic imaging is required. A need for an X-ray system could also arise in ambulance cars and services for nursing homes. In some scenarios where X-ray systems are used an optimized operation of such systems seems desirable. Furthermore, all safety requirements for the operation of such systems must be fulfilled.

### SUMMARY OF THE INVENTION

There may thus be a need to improve the access and operation control of X-ray systems.
The object of the present invention is solved by the subject-matter of the independent claims; further embodiments are incorporated in the dependent claims. It should be noted that the following described aspects of the invention apply also for the X-ray device, the X-ray system as well as for the described method for image acquisition by use of the X-ray system and vice versa.

According to at least some examples, the features provided by the invention as described in the following are also particularly for a mobile X-ray device, a mobile X-ray system and a method of operation thereof. A mobile X-ray device, when used in rural areas or nursing homes, is brought to the patient for scanning procedures. Furthermore, at least some features of the invention presented herein also apply to other mobile X-ray health systems.

Generally, untrained or uninformed operators of a medical device usually need guidance on how to operate it. This also holds true for X-ray systems and devices, particularly when they are operated in decentralised areas such as rural areas and are mobile X-ray systems and devices. An easy step-by-step way of operation can be expected to be followed by less trained or less informed operators particularly when using mobile X-ray systems or devices.

Well trained medical personnel, however, might experience such guidance as annoying and less helpful for cases which they can easily and quickly fulfil based on their knowledge of the required workflow and parameter settings. In addition, well trained medical personnel may have additional knowledge compared to what a standard workflow would provide.

According to the present invention, an X-ray device is provided which supports an operator using the system in an appropriate manner according to the operator expertise level. The X-ray device comprises an X-ray source, a detector, an ID-system comprising one or more ID-means for identifying at least one operator and/or at least one object, a processor unit and an interface for adaptive interaction of the operator and X-ray device. The processor unit comprises determining an operator expertise level and/or determining object data and image acquisition for generating an X-ray image. The interface is adapted for outputting at least one option selector and at least one subsequent option selector is adapted according to the operator expertise level and/or object data. Thus, the invention proposes how to cope with X-ray devices and systems as well as operators or to be scanned objects in decentralized areas, so that a safe operation is ensured. Furthermore, according the invention the way of operation is adapted to the situation on-site, which also depends on the operator expertise level.

The ID-means provided by the X-ray device identify whether the operator is known to the X-ray device by comparing operator details, such as name, registration number etc. to data provided by the X-ray device. The ID-means further comprise identification of the information about the operator's expertise and/or object data. The object is a patient or another object subject to X-ray image acquisition. Based on this information the way of operation of the X-ray device is modified or adapted in such a way that the operator expertise level matches at least one option selector which is outputted or provided by the interface.

The at least one or subsequent option selector can also be adapted or modified according to the identified object data. The outputted option selector is selectable via the interface for adaptive interaction of the operator and the X-ray device. The interface for adaptive interaction comprises at least one of a display, a touch-screen, keyboard and the like for entering data and for displaying information.

The at least one or subsequent option selector comprises one or more of the following: dose settings or maximum dose settings depending on the object (e.g. patient size, patient weight), number of allowed X-ray scans per object.

The outputted option selector can thus provide more freedom of choice for an operator expert to select protocols, for example, while a non-expert operator could perform the same or a similar way of operation by following a step-by-step guidance provided by the at least one option selector. Accordingly, at least one subsequent option selector is adapted and the at least one outputted option selector is updated via the interface for further adaptive interaction of the operator and the X-ray device before the actual generation of an X-ray image (image acquisition) and outputting the generated X-ray image.

According to an example, the at least one option selector and/or the at least one subsequent option selector provides a way of operation and/or a guidance level. The X-ray device provides adaptation of subsequent option selectors according to the operator expertise level and/or object data, so that the interface is adapted for outputting option selectors for providing the needed or required guidance for the operator. At the same time the way of operation of the system is also adapted according to the operator's request. In some examples, the operator might request guidance or way of operation as for a less qualified operator. Such requests could be provided via the interface for adaptive interaction of the X-ray device.

According to an aspect, an X-ray system is provided comprising an X-ray device. Adaptive interaction of an operator and the X-ray device is provided by an interface adapted for outputting at least one option selector. Accordingly, at least one subsequent option selector is adapted according to the operator expertise level and/or object data.

According to an example, the X-ray system further comprises at least one sensor for detecting an object and/or an operator of the X-ray system. The data received by the sensor can be provided to the operator by the at least one or subsequent option selector for guidance of the operator. The sensor data can be also used for double checking the settings of the X-ray device or system. In some examples the X-ray system comprises a sensor system.

According to an example, the at least one sensor is a vicinity sensor for sensing the distance of the operator and/or the object from the X-ray device. Vicinity sensors can be used for sensing the absence of another object or person near the X-ray device, so that safety protection against unintentional X-ray radiation is provided.

According to an example, the X-ray system is connected to a cloud based infrastructure providing operator expertise level information and/or object data. The cloud based infrastructure is updated in an online or offline mode or synchronized with the expertise data provided on an identification device of the operator. Also, the object data are updated in the same way. Thus, the operator expertise level and/or object data is always up-to-date.

According to an aspect, a method for image acquisition by use of an X-ray system is provided, wherein the X-ray system comprises an X-ray device. The method comprises determining an operator expertise level and/or determining object data. The method further comprises outputting at least one option selector according to the operator expertise level and/or object data and selecting at least one option selector. At least one subsequent option selector is adapted according to the operator expertise level and/or object data. These steps are performed and/or repeated prior to generating an X-ray image and outputting the generated X-ray image.

According to the invention, the at least one and/or subsequent option selector are matching an expertise level of the operator, so that a highly skilled operator can interact with the X-ray system in a more flexible manner as it is required for a low skilled operator. Thus, a flexible method is provided, which provides an adapted way of operation and guidance simultaneously.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 an X-ray system according to the invention;
Fig. 2 a method for image acquisition according to the invention - basic operator expertise level;
Fig. 3 a method for image acquisition according to the invention - expert operator expertise level.

### DETAILED DESCRIPTION OF EMBODIMENTS

This invention generally relates to an X-ray system and X-ray device, or a mobile X-ray system and mobile X-ray device, and a method of operation thereof. An X-ray device is provided which supports an operator using the system in an appropriate manner according to the operator expertise level. Operator related information, such as medical qualifications due to professional qualification, courses, trainings and the like, or on the other hand, less or no expertise, is stored along with data for follow-up as well as for selection of an operator expertise level of interaction and/or operation of the X-ray system. Stored follow-up data comprises for example procedures after a first diagnostic step or treatment, such as a rescan for control of the actual scan, storage of the data in a personal health record of a patient and the like.

Herein, level of interaction and/or operation can be indicated by an option selector adapted to a basic or expert mode. In addition to identification data of the operator also information about a training level of the operator is provided. Based thereon an adapted selection of settings and guidance by at least one option selector during the operation of the X-ray system is provided. Such adaptive interaction of the X-ray system with the operator ensures good operation quality and operator guidance whenever necessary.

The outputted option selector can thus provide more freedom of choice for an operator expert to select protocols, for example, while a non-expert operator could perform the same or a similar way of operation only by following a step-by-step guidance provided by the at least one option selector. Protocols are scanning protocols: e.g. for pediatric, for cardiac, or selection of small region of interest. Generally, such protocols comprise predefined parameter settings according to a disease or region. Also, the current and voltage range of the X-ray device can be adapted, e.g. range of mA or kV, respectively (e.g. an adult's protocol with high mA is not used for a child, to prohibit overdosing).

According to an example of the invention, the at least one option selector and/or the at least one subsequent option selector is provided in a step-by-step mode, if the operator expertise level is identified as less or low experienced as described above. The step-by-step mode can comprise specific double-check questions such as request for confirmation of dose settings or re-entering the age of a patient, or double checking the position of the object, entering data, push button confirmation, automatic checks of cameras provided at the X-ray device or X-ray system, automatic checks of sensors measuring the geometry of the object (patient) for double check of settings provided as input by the operator during the adaptive interaction with the X-ray device via the interface.

The size of the object (patient) or its diameter determines the X-ray dose (i.e. setting of current and voltage in a mA or kV range, respectively) necessary to get a good image quality with low noise. Thus, the expected range of voltage and current should match the "length of tissue", which is the passing length for X-ray photons from the X-ray source to the detector of the X-ray device. Such derived length from the parameter setting could be used for parameter setting check. Also, a rescan should only be possible, if a first generated X-ray image does not meet the quality criteria. Also, if any other additional information is missing which is required, a rescan should be possible if regulation allows.

In a step-by-step mode, additional confirmation procedures can be included in the way of operation and/or guidance level provided to the operator. In some examples, a step-by-step mode is also provided if the operator expertise level is identified as well trained. This might be the case, if a provided input required by an option selector is out of range or does not match the object data, e.g. the dose setting for a child is required, but the dose setting for an adult is given as input, or the number of scans exceeds the highest allowed number for a particular patient to be scanned, or the input current and voltage range would lead to an overdose. In some examples, other sensor systems could also be provided for supporting such automated checks. However, the level of automatic procedures and sensors might also depend on a cost-target for the X-ray system.

According to some examples, at least one subsequent option selector is optional according to the operator experience level. This provides a fast way of operation, if the operator expertise level is appropriate. An optional option selector can also provide the same level of guidance as for a less skilled operator or an upgraded level of guidance as for a more experienced operator. This optional option selector provides high flexibility in such cases where the operator expertise level is identified as high, however the operator itself might want additional guidance, as the expertise level has been acquired in a particular field or if the operator faces some features, e.g. from the treatment history of a patient, which needs more support by the device or system.

According to some examples, the selected option selector is subject of consistency checks prior to adapting at least one subsequent option selector. Such consistency checks could also depend on the treatment history of the patient, the age or prior number of scans on-site. In an example, consistency checks are performed for crucial parameter settings, such as dose settings, or according to the operator expertise level to ensure safe operation of the system.

In some examples, the systems and devices, as well as their method of operation proposed by the invention also provide overruling of such measures, if an appropriate operator expertise level is identified. Accordingly, some of the examples provide overruling or waiving of one or more of the at least one option selector or of one or more subsequent option selectors according to the operator expertise level. Only an operator with an identified high expertise level can waive outputted option selector such as warnings, for example.

In some examples, the operator expertise level and/or object are to be confirmed, if a sensor of the X-ray system detects an absence of the operator and/or object. This might be the case, if the object moves out of the detection area or there is a need for the operator to interrupt the operation of the X-ray device. In some examples, such measures are linked to the identified operator expertise level.

According to an example, the X-ray system is connected to a training or expert level database infrastructure, cloud based infrastructure, which enables a wider usage also for other imaging or healthcare service modalities.

Operator expertise level can be provided by a database. Such database could be a cloud service and/or a local application on a mobile phone. The database providing the qualification skills or expertise level of the operator could be a cloud based infrastructure that provides local copies for individual operators on the identification device which interacts with the ID-means. Such copy can be provided as a mobile phone app, a health card, etc. to enable also offline operation. Also, each suitable device which can be operated without a network but providing bilateral communication between the identification device and the X-ray device. The data for the operator expertise level could be linked to training systems or other sources of relevant information such as professional data from hospitals or other education centers.

For the identification of the operator an easy and fast working procedure is desirable. Some mobile phones are equipped with NFC (near field communication) modules that are normally used for payment. Such technology is suitable, but also Bluetooth or other RF links of the mobile phone could be used to interact with the X-ray device, as long as the identification of the operator can be detected unambiguously. Also, a health card, ID card or even passport could be used for identification.

In addition to the mobile phone identification or other ways of identification as above, a list of qualified operators could be checked online/offline via the X-ray system to give additional access rights to the system. E.g. a physician or well-trained person might have more options to operate the system, so that an operator's expertise level could be changed on-site.

Another possible option to handle information exchange could be a mobile phone app that stores locally (updated via database in network/cloud, ...) on a mobile phone of the individual operator medical qualifications of the operator due to professional qualification, courses, trainings, etc. or no expertise (which would be a default setting). A qualification acquired by an online course could be automatically stored in the database and transferred to the mobile device.

Similar identification could be performed for the object data, e.g. a patient identification or treatment history, to get the appropriate data/information from a patient mobile phone or health card, ID card or passport, as long as information exchange can be performed.

In some examples a link to the training and expert certification database is provided, which allows a supplier independent platform technology.

In some examples the X-ray system also provides disqualification of an operator or an expertise level of the operator is downgraded or at least questioned, if automatic consistency checks fail. However, option selectors are provided to overrule such checks, if the operator is a highly-qualified expert, e.g. a physician having long term expertise.

The operator or object data are determined by identifying the operator and/or object via an identification device, such as a mobile phone, ID card or the like. Identification devices are configured to communicate or interact with the ID-means of the X-ray device. The identification devices and/or X-ray device can be connected to a network or work offline. In some examples the identification devices are part of the X-ray system o X-ray device; they may be incorporated or independent of the system or device. Based on the selected option selector, at least one subsequent option selector is adapted according to the operator expertise level and the outputted at least one option selector is updated.

The method according to the invention provides that at least one subsequent option selector is adapted according to the operator expertise level and/or object data. By this, a flexible way of operation is provided which is adapted to the skill and knowledge of the operator and/or the identified object data. The outputted option selectors also provide guidance for the operator matching the operator's expertise level. In some examples a highly skilled operator might select an option selector, so that the method provides guidance and/or way of operation of a lower skilled operator. However, all operator expertise levels are provided by the X-ray system and cannot be entered by a user, except for such operators which are admitted by the X-ray system to register a new user and provide the required information about the expertise level.

For all examples, documentation of the steps, parameters and decisions is performed including the adaptation of the operation selectors and the outcome of the image acquisition. This provides necessary data for a follow-up of the patient data. Also the operator expertise level for a particular operator can be addressed.

The features of the invention are described using an X-ray system. It should be noted that the following description of the figures is also for a mobile X-ray device, mobile X-ray system or for other mobile health systems or devices.

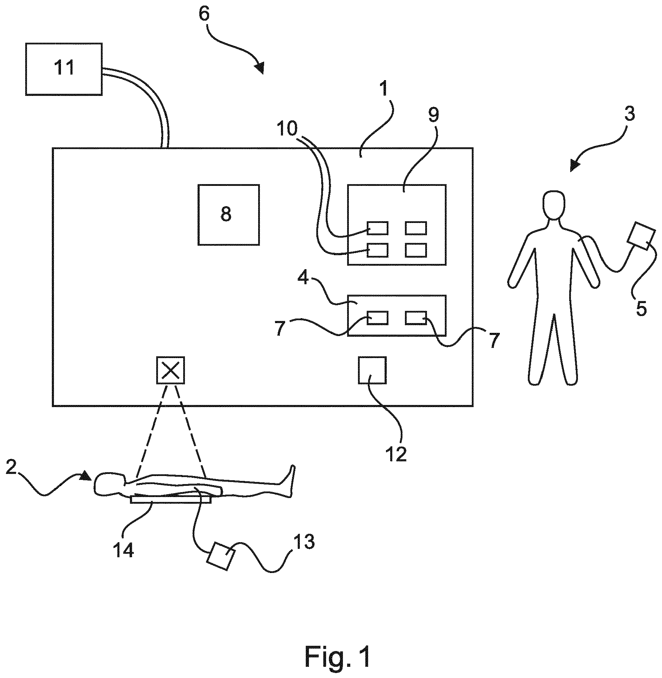

Fig. 1 shows an X-ray system 6 comprising an X-ray device 1 and an optional sensor 12 for detecting an object 2 to be scanned or an operator 3 of the X-ray system. The object 2 is arranged between the X-ray source and the detector 14. The sensor 12 could also comprise a sensor system comprising at least two sensors for detecting the distance of the object 2 from the X-ray device or the X-ray source X of the X-ray device 1 or the operator 3. In some embodiments, the sensor(s) 12 is (are) a vicinity sensor(s) for detecting the absence of other objects or people in the vicinity of the X-ray device.

The X-ray device 1 further comprises an ID-system 4 with four ID-means 7 for identification of the operator 3 and the object 2. The operator is identified via an operator identification device 5 and the object is identified via an object identification device 13. More than one ID-means 7 can be provided to reflect the multiple ways of identification devices which can be detected by the ID-system 4. In this embodiment, the operator identification device 5 has an RFID chip and the identification is performed via a RF link to the ID-system of the X-ray device 1. The identification device 13 of the patient (object 2) is a mobile phone in this embodiment. The identification can be performed via the phone number, IMEI or finger-print identification provided by the mobile phone.

Operator and patient (object) identification are performed via technology platforms available in the vicinity of the location where the X-ray system 6 is used. Typical identification devices suitable for identification of the operator 3 or patient (object) 2 are mobile phone, e-health card, ID card with RF link, electronically readable passport.

The provided ID-system 4 of the X-ray device 1 can be designed to reflect the expected scenario on-site or dependent on the available technology in the country of use. In any case, the ID-means 7 of the IS-system 4 should be available to most of the people (operator 3, patient 2) involved in the image acquisition. Possible identification devices 5, 13 can be provided for each or some of the embodiments: mobile phone, including an application (also called App); ID card; e-health card (with NFC); camera with face recognition, wherein online database comparison should be available; RF ID tag; personalized watch and the like. In hospitals, nursing home or any other professional environment the staff normally has an ID card providing a code. The ID-means 7 can be configured to read such code. Depending on GSM connection online/offline data is provided by the ID-system. In this embodiment, a database 11 provides data relating to the training level of the operator 3.

The ID-system 4 and the ID-means are adaptable to any available technology for identification via interaction with the X-ray system 6.

The X-ray device 1 further comprises a processor unit 8. The processor unit 8 is configured to determine an operator expertise level as expert level E or basic level B by matching the identification information received from the ID-system 4 and the information provided by the database 11. In some embodiments, intermediate levels between the expert level E and the basic level B are provided according to the identified medical qualifications due to professional qualification, courses, trainings and the like of the operator. These intermediate levels could be a sub-level of the basic level, for example, including the requirements for basic level and intensive training in one medical field, or a sub-level of the expert level, including the requirements of the expert level and long-term expertise, for example. In some embodiments, the operator expertise levels can be configured including operator expertise level for specific applications, so that the operator expertise level can be changed during an operation if such specific applications are to be used. Also, the operator expertise level can be configured to include identification of having no expertise in some fields of operation. In some embodiments, an operator 3 may be admitted registering a new operator via the ID-system 4. In such embodiments, the ID-system 4, the processor unit 8 and/or an interface 9 is configured to perform such registration by providing according output and receiving according input.

The processor unit 8 checks whether the operator 3 is known to the X-ray system 6 as provided in the database 11. The database 11 could also be integrated in the X-ray device 1. The processor unit 8 also checks the patient identification if available in the database 11. If available the patient 2 treatment history can be provided to the processor unit 8 via the database 11.

Accordingly, an operator expertise level B, E is determined and the interface 9 for adaptive interaction of the operator 3 and the X-ray system 1 is provided. The interface 9 is configured to output at least one option selector 10, however the number of option selector 10 is adapted according to the required guidance of the operator 3 depending on the identified operator expertise level. Via the interface 9 the operator 3 can select or even waive the outputted option selector 10, which will be explained in more detail with reference to the other figures.

The operator 3 performs image acquisition by activating the X-ray source X of the X-ray device 1 after all required steps are performed/confirmed by interaction with the X-ray device 1 via the interface 9.

As will be explained in more detail, the option selector 10 of the interface 9 are adapted, so that an operator 3 can perform image acquisition adapted to its expertise level. This provides a highly flexible X-ray system 6 which can be used in cases such as in a rural kiosk, in a nursing home or in a hospital or other location with multiple users (professional care). If the "expert level database" is available via a mobile phone app or identification link the provided X-ray system could be used also in scenarios for identifying or checking a physician's expertise level in a remote location such as a plane for example, in case there is doubt about the expertise of such person to ensure that only trained personnel treats patients.

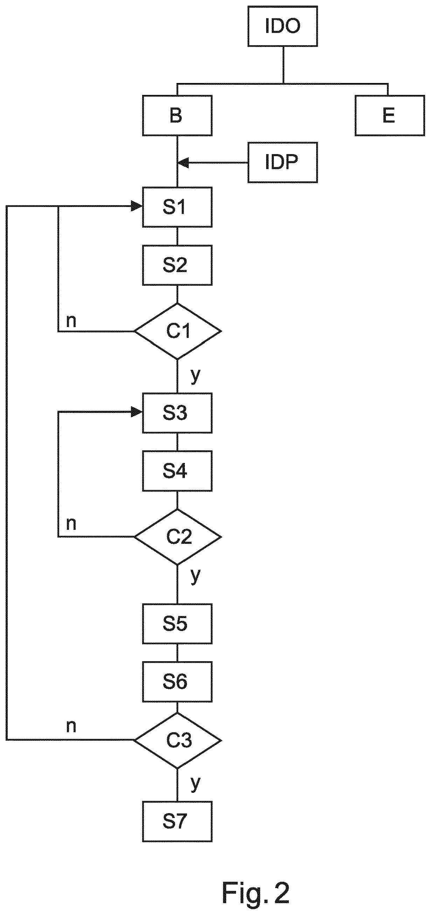

Fig. 2 shows a method for image acquisition according to the invention, wherein the operator expertise level is identified as basic B. An identified operator is familiar with the X-ray system 6. An operator is not admitted to operation of the X-ray system 6 or X-ray device 1, if the operator is not familiar with the system. In step IDO identification of the operator is performed by using the ID-means 7 and the identification devices 5, 13 as described in Fig. 1. Accordingly, in the next step the processor unit 8 determines a basic operator expertise level B and other operator expertise levels such as expert level E or operator expertise level for specific applications or operator expertise level having no expertise in some fields of operation.

In this embodiment, the identified operator expertise level is basic and no expert level E has been identified. The basic operator expertise level B is also a default setting which is provided in all embodiments. In cases where communication via the ID-system 4 might fail for whatever reason, the operator expertise level is set to basic B. In some embodiments, no operation is allowed unless in urgency cases, wherein maximum one X-ray scan along with detailed guidance with option selectors in a step-by-step mode is allowed. Such scan is documented and stored in the database for follow-up.

In step IDP identification of at least one object 2 is performed. In this embodiment the object 2, which is a patient, e.g. a child, is already known to the X-ray system 6 and treatment history data, personal data such as age, size, weight is already provided by the database 11. In other embodiments, the patient is not known to the X-ray system 6.

In the next step S1 at least one option selector 10 is outputted via the interface 10 of the X-ray device 1. In this embodiment, the outputted option selector 10 comprises all relevant basic information shown on a display of the interface 9, which also corresponds to the default setting. Option selector 10 relating to dose settings provides only basic settings with low (radiation) risk. As the operator expertise level is identified as basic B also, double checks of positions and setting by additional questions and/or sensors or consistency checks are performed before a subsequent option selector 10 is adapted, as explained in the following.

A basic expertise level B includes basic knowledge and basic training. Also, in this embodiment a short information summary update is given According to the identified operator expertise level B simplified consistency checks are performed. At such basic expertise level B a step-by-step mode is provided by the option selectors which guides the operator through the way of operation until the image acquisition is performed.

In step S1 at least one option selector provides a dose setting request. The operator interacts with the interface by selecting this option selector by inputting a number for the dose setting at step S2. The system then performs an option selector as consistency check C1, to check whether the dose setting is according to the patient data, a child in this embodiment. If the child was subject to image acquisition lately only a certain dose is allowed or the age/size of the child requires a certain dose setting. In case the dose setting is appropriate the next step S3 is performed. If the dose setting is not appropriate, the operator is requested to select a new dose setting, starting again with step S1. Optionally, the operator might be guided by additional information displayed by option selector. In some embodiments, the consistency check C1 is not provided or is optionally provided.

In step S3 the subsequent option selector is adapted according to the expertise level basic B and outputted via the interface by updating the option selector. The subsequent option selector is a request for the operator to check whether the patient (object) is in a good condition and aware of the following X-ray operation. The operator must confirm by selecting this option selector.

The next step S4 requires an input of the distance between the object and the X-ray source X. The operator selects this option selector by inputting the distance at step S4. An option selector as confirmation check C2 is performed, as this embodiment the distance is also measured by a sensor of the X-ray system 6. If the values are consistent, the subsequent option selector is adapted and the option selector is updated via the interface at the next step S5. If the values differ, the subsequent option selector is adapted in such a way that the option selector is updated via the interface at step S3 and this option selector are selected again.

At step S5 an option selector for generating an X-ray image is provided, so that image acquisition is performed by selecting this option selector. The generated X-ray image is displayed via the interface at step S6.

The subsequent option selector is performed as consistency check C3, whether the image can be used for diagnosis. If yes, image acquisition stops at step S7 by updating the option selector by the adapted subsequent option selector indicating the end of image acquisition. If the image is not usable, the procedure starts all over at step S1 by adapting a subsequent option selector and updating the option selector via the interface, so that dose setting might be provided again.

The described option selector and the adaption of the subsequent option selector according to the selected option selector, also by confirming or entering values, are only exemplary and can also include other measures in other embodiments.

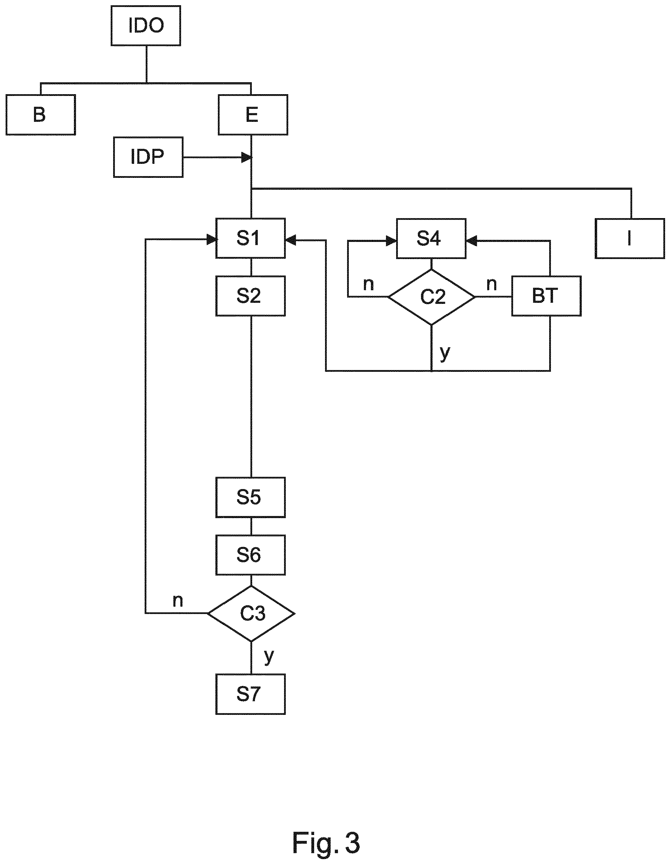

In Fig. 3 a method for image acquisition according to the invention. In this embodiment, an expert operator expertise level E is determined after operator identification in step IDO. According to determined patient (object) data, which is a child, at step S1 at least one option selector provides a dose setting request. The operator with expert expertise level E interacts with the interface by selecting this option selector by inputting a number for the dose setting at step S2. As the expertise level is determined as expert level E, the dose setting is checked and the operation can proceed without being blocked, even if the dose setting might be regarded as not acceptable according to the identified object data.

This means, the subsequent option selector is adapted as image acquisition at step S5 and an option selector for generating an X-ray image is provided. Image acquisition is performed by selecting this option selector. The generated X-ray image is displayed via the interface. As for the basic operator expertise level B, the subsequent option selector is performed as consistency check, whether the image can be used for diagnosis. If yes, image acquisition stops at step S7 by updating the option selector by the adapted subsequent option selector indicating the end of image acquisition. If the image is not usable, the procedure starts all over at step S1 by adapting a subsequent option selector and updating the option selector via the interface, so that dose setting might be provided again.

However, the method provides optionally option selector at step 4 as input of the distance between the object and the X-ray source X. The operator can select this option selector by inputting the distance at step S4 or can immediately start by selecting the option selector provided at step S1. If the option selector at step 4 is selected an option selector as confirmation check C2 is performed, as this embodiment the distance is also measured by a sensor of the X-ray system 6. If the values are consistent, the subsequent option selector is adapted and the option selector is updated via the interface at the next step S1. However, if the check is not consistent the subsequent means is adapted as selection button BT which provides an option selector for waiving the inconsistency. Such selection is only provided at expert expertise level E. If the button BT is selected the subsequent means is adapted as dose setting at step S1 or distance input at step S4. Both option selectors are provided via the interface by updating the option selector. It should be noted that additional option selector at step I provide additional options, if required. Such option selectors are not provided to the default operator or the basic operator.

For the expert operator required information can be provided without detailed explanation as option selector. This ensures a fast update of the operator. Also, a full range of parameter selection is allowable and provided by the option selector. Option selector such as consistency checks are reduced, however, the expert operator can select additional option selector if deemed necessary.

The adaptation of the option selector to the operator expertise level provides a highly flexible and fast way of operation of the X-ray system. In addition, also the guidance for the operator is adapted to its expertise level.

It should be noted that also other embodiments can be provided, wherein the option selector at the different steps as described above comprise one or more of the following: number of X-ray scans per object, alignment of the object and an X-ray source of the X-ray device, push button confirmation, and automatic checks. Accordingly, the option selectors are adapted to the expertise level of the operator.

In another embodiment the option selector can include a confirmation of the operator expertise level and/or object at step IDO, if a sensor the X-ray system detects an absence of the operator and/or object for a longer time. Generally, the described X-ray device and system and the operation thereof allow one scan per object (patient) if there is no specific reason for a rescan which has to be confirmed by an expert. If such second scan is necessary, the system checks the identity of the patient. If the patient leaves the scan area, for whatever reason, and a second patient enters the scan area, the system allows the second scan. However, the operator is warned by the device providing an option selector via the interface. Accordingly, the data of the new patient is updated or edited. In case the first already scanned patient re-enters the scan area, the system identifies the patient as the object scanned before or at least gives information by outputting an option selector that the object has been replaced by the same or another person.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It should be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray device, comprising:
- an X-ray source;
a detector;
- an ID-system comprising one or more ID-means for identifying at least one operator and/or at least one object;
- a processor unit comprising:
- determining an operator expertise level and/or determining object data;
- image acquisition for generating an X-ray image; and
- an interface for adaptive interaction of the operator and X-ray device, wherein the interface is adapted for outputting at least one option selector and wherein at least one subsequent option selector is adapted according to the operator expertise level and/or object data.

2. An X-ray system, comprising:
- an X-ray device as in claim 1;
- wherein an adaptive interaction of an operator and the X-ray device is provided by an interface adapted for outputting at least one option selector and wherein at least one subsequent option selector is adapted according to the operator expertise level and/or object data.

3. System according to claim 2, further comprising at least one sensor for detecting an object and/or an operator of the X-ray system.

4. System according to claim 3, wherein the at least one sensor is a vicinity sensor.

5. System according to claim 2, wherein the system is connected to a cloud based infrastructure providing operator expertise level information and/or object data.

6. System according to claim 5, wherein the database is updated in an online or offline mode or is synchronized with an identification device.

7. A method for image acquisition by use of an X-ray system, wherein the X-ray system comprises an X-ray device, the method comprising the following steps:
a) determining an operator expertise level and/or determining object data;
b) outputting at least one option selector according to the operator expertise level and/or object data;
c) selecting at least one option selector;
d) adapting at least one subsequent option selector;
e) updating the outputted at least one option selector by at least one subsequent option selector;
wherein at least one subsequent option selector is adapted according to the operator expertise level and/or object data.

8. Method according to claim 7, wherein at least one subsequent option selector is provided in a step-by-step mode.

9. Method according to claim 7, wherein at least one subsequent option selector is an optional option selector according to the operator experience level.

10. Method according to claim 7, wherein the selected option selector is subject of consistency checks prior to adapting at least one subsequent option selector.

11. Method according to one of the claims 7 to 10, wherein one or more of the at least one option selector can be waived according to the operator level experience.

12. Method according to claim 7 to 11, wherein the operator expertise level and/or object are to be confirmed, if a sensor the X-ray system detects an absence of the operator and/or object.

13. Method according to claim 7 to 12, wherein the option selector comprises dose setting, number of X-ray scans per object, alignment of the object and an X-ray source of the X-ray device, push button confirmation, automatic checks.

14. A computer program element for controlling an apparatus according to one of the claims 1 to 6, which, when being executed by a processing unit, is adapted to perform the method steps of one of the claims 7 to 13.

15. A computer readable medium having stored the program element of claim 14.
